# EUROPEAN PATENT APPLICATION

(11) **EP 2 322 528 A1**
(43) Date of publication of application: **18.05.2011**
(21) Application number: 08874713.4
(22) Date of filing: 18.12.2008
(51) Int. Cl.: C07D 487/04, A61K 31/55, A61P 35/00, C07D 491/20

(54) **STEMONAMIDE SYNTHESIS INTERMEDIATE AND PHARMACEUTICAL COMPOSITION FOR PREVENTION AND/OR TREATMENT OF CANCER**

(30) Priority: 19.06.2008 JP 2008160878
(71) Applicant: National University Corporation Kanazawa University, Kanazawa-shi, Ishikawa 920-1164 (JP)
(72) Inventor: MUKAIDA, Naofumi, Kanazawa-shi Ishikawa 920-1164 (JP); ISHIBASHI, Hiroyuki, Kanazawa-shi Ishikawa 920-1164 (JP); TANIGUCHI, Tsuyoshi, Kanazawa-shi Ishikawa 920-1164 (JP)
(74) Representative: HOFFMANN EITLE
(86) International application number: PCT/JP2008/073094
(87) International publication number: WO 2009/153897

(57) **Abstract**

An objective of the invention is to provide a compound effective for prevention and/or treatment of cancer. The invention relates to a compound according to by formula I, or a salt, solvate or physiologically functional derivative thereof, and a composition for prevention and/or treatment of cancer comprising the same as an active ingredient: 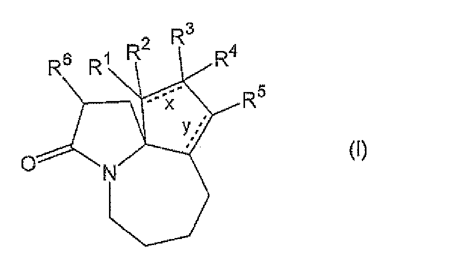 wherein R¹ to R⁶, x, and y are as defined in the description.

## Description

### Technical Field

The present invention relates to a stemonamide synthesis intermediate and a pharmaceutical composition for prevention and/or treatment of cancer.

### Background Art

Along with advancements in elucidation of the molecular mechanisms of carcinogenesis, development of so-called molecular-targeted agents, which target molecules that are closely involved in the carcinogenic mechanism, and signal transduction molecules in particular, has been actively attempted in recent years. While the expression of signal transduction molecules that can be targeted by molecular-targeted agents is hardly observed in normal cells, the expression thereof is elevated in cancer cells, and such signal transduction molecules play an essential role during the process of cancer cell growth. Molecular-targeted agents that have heretofore been developed target growth factor receptors fulfilling the aforementioned conditions or tyrosine kinase located at a site downstream thereof.

The present inventors analyzed gene expression patterns in mouse models of liver cancer, and they demonstrated that expression of Pim-3 (i.e., a proto-oncogene, the expression of which is not observed in normal liver tissue, having serine/threonine kinase activity) was elevated at the stage of precancerous lesion and thereafter in a mouse liver and similarly elevated Pim-3 expression was observed in a human liver cancer lesion (Int. J. Cancer 114: 209-218, 2005). Further, suppression of Pim-3 expression in the human liver cancer cell line via RNAi delays the cell-cycle progression involving increased apoptosis. Based thereon, the present inventors assummed the possibility of the involvement of Pim-3 with the cell growth process in the liver cancer cell line. At this time, the entire nucleotide sequence of human Prim-3 cDNA, which had not previously been determined, was determined.

As in the case of the liver, Pim-3 proteins are not detected in a normal human pancreas, large intestine, or stomach, all of which are endoderm-derived organs. However, the level of Pim-3 protein expression is elevated in about 50% of colon cancer, gastric cancer, and liver cancer cases and all pancreatic cancer cases. In such cancer cell lines, suppression of Pim-3 expression was found to delay the cell cycle progression involving increased apoptosis (Cancer Res. 66: 6741-6747, 2006; Cancer Sci. 98 : 321-328, 2007). The above results indicate that Pim-3 is involved in the carcinogenic process for cancers of endoderm-derived organs, and pancreatic cancer in particular. The results also indicate that Pim-3 is a target molecule when treating such cancer. It was also demonstrated that Pim-3 selectively phosphorylated and inactivated the serine residue 112 of the proapoptotic molecule Bad to inhibit apoptosis (Cancer Res. 66: 6741-6747, 2006; Cancer Sci. 98 : 321-328, 2007).

It is reported that Prim-1 and Pim-2 are members of the Pim family and they both phosphorylate the serine residue 112 of Bad. Further, the analysis of the entire nucleotide sequence of cDNA demonstrates that Pim-1 is highly homologous to Pim-3, including with respect to the active center. While the increased expression level of Pim-1 in malignant tumors of hematopoietic organs and that of Pim-2 in prostate cancer have been reported, there have not been any reports that the expression level of Pim-1 and of Pim-2 are elevated in cancers of endoderm-derived organs, or in pancreatic cancer in particular.

Stemona alkaloids, such as stemonamide, isostemonamide, stemonamine, and isostemonamine, isolated from *Stemona japonica* are used for cough medicine in folk remedies in China and in Japan, and they are also used for insecticides (Nat. Prod. Rep. 2000, 17, 11.7; J. Nat. Prod. 1994, 57, 665). However, other pharmacological activities of stemona alkaloids have not been known. Regarding stemonamide and isostemonamide, Kende et al. succeeded in synthesis thereof for the first time (Org. Lett. 2001, 3, 2505), and Tu et al. succeeded in synthesis of stemonamine for the first time (Org, Lett. 2008, 10, 1763).

### Summary of the Invention

The present invention provides a compound effective for prevention and/or treatment of cancer.

The present inventors studied the influence of the synthesized candidate compounds on Pim-3 activity via enzyme immunological techniques. As a result, they discovered compounds that would significantly inhibit Pim-3 activity, and they further discovered that such compounds would suppress growth of cancer cell lines *in vitro,* thereby completing the present invention.

Specifically, the present invention include the followings.
(1) A compound according to formula I: 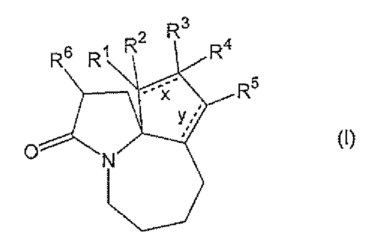 wherein
   R¹ and R² are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R³OR^{d}, -NR^{b}R^{c}, -R^{a}NR^{b}R^{c}, -R^{a}C(O)R^{d}, -C(O)R^{d}, -CO₂R^{d}, -R³CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
   R¹ and R², together with the carbon atom to which they are attached, may form an optionally substituted 4-, 5-, or 6-membered ring or a carbonyl group (>C=O);
   R³ and R⁴ are independently H, halogen, C₁₋₆ haloalkyl, C₁₋₆ alkyl, C₂₋₆ alkenyl, C₂₋₆ alkynyl, C₃₋₁₀ cycloaakyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
   R³ and R⁴ may together form =CH-(CH₂)ₙ-Ar, =CH-R^{a}Ar, =CH-(CH₂)ₙ-Het, =CH-R^{a}Het, or an oxo group (=O);
   R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
   a broken line indicates that a double bond may be present and, when a double bond represented by x is present, either R¹ or R² and either R³ or R⁴ are absent;
   n is independently 0, 1, or 2;
   m is independently 0, 1, or 2;
   R^{a} is independently C₁₋₆ alkylene, C₃₋₁₀ cycloalkylene, C₂₋₆ alkenylene, C₃₋₁₀ cycloalkenylene, or C₂₋₆ alkynylene;
   R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R^{a}C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)ₘR^{d};
   R^{d} is independently H, C₁₋₆ alkyl, C₂₋₆ alkenyl, C₂₋₆ alkynyl, C₃₋₁₀ cycloalkyl, or -Ar; R^{e} and R^{f} are independently H or C₁₋₆ alkyl;
   Ar is independently optionally substituted aryl; and
   Het is independently an optionally substituted 4-, 5-, or 6-membered heterocyclic group,
   except for a case in which R¹ and R², together with the carbon atom to which they are attached, form a 5-membered ring according to the following formula: 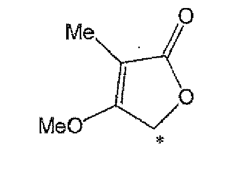 wherein * represents the carbon to which R¹ and R² are attached, R³ and R⁴ together form an oxo group (=O), R⁵ is a methyl group, a double bond represented by y is present, and R⁶ is H, or a salt, solvate or physiologically functional derivative thereof.
(2) The compound according to (1) or a salt, solvate or physiologically functional derivative thereof,
   wherein
   R¹ is OH or H and R² is -R^{a}CO₂R^{d} or
   R¹ and R², together with the carbon atom to which they are attached, a carbonyl group (>C=O) or a 5-membered ring according to formula II: 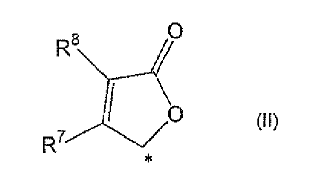 wherein R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
   * represents the carbon to which R¹ and R² are attached;
   R³ and R⁴ are both H, or
   R³ and R⁴ together form =CH-(CH₂)ₙ-Ar or an oxo group (=O),
   provided that, when a double bond represented by x is present, R¹ and R³ are absent;
   R⁵ is H or C₁₋₆ alkyl;
   R⁶ is H; and
   R^{a} to R^{d}, n, and m are as defined in (1).
(3) The compound according to (2) or a salt, solvate or physiologically functional derivative thereof,
   wherein
   R¹ is OH or H and R² is -C ≡C-CO₂-C₁₋₆ alkyl, or
   R¹ and R², together with the carbon atom to which they are attached, form a carbonyl group (>C=O) or a 5-membered ring according to formula II: 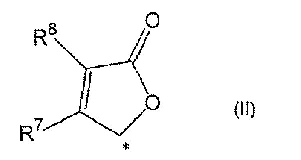 wherein
   R⁷ is C₁₋₆ alkoxy and R⁸ is C₁₋₆ alkyl or halogen;
   R³ and R⁴ are both H, or
   R³ and R⁴ together form =CH-phenyl, =CH-halophenyl, or an oxo group (=O), provided that, when a double bond represented by x is present, R¹ and R³ are absent;
   R⁵ is H or C₁₋₆ alkyl;
   R⁶ is H; and
   R^{a} to R^{d} and m are as defined in (1).
(4) A compound selected from the group consisting of the compounds below or a salt, solvate or physiologically functional derivative thereof. 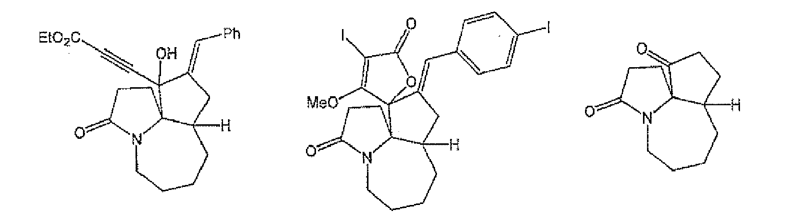 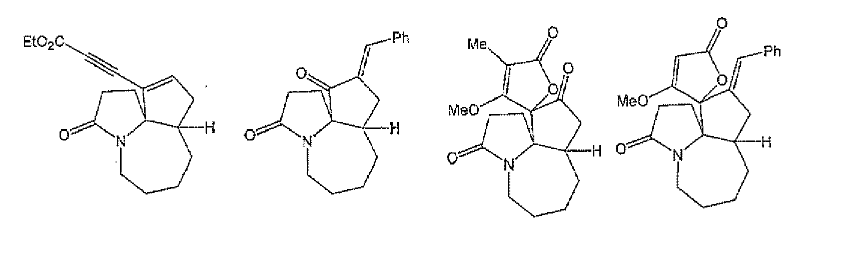
(5) A pharmaceutical composition comprising the compound according to any one of (1) to (4) or a salt, solvate or physiologically functional derivative thereof.
(6) A pharmaceutical composition for prevention and/or treatment of cancer, which comprises, as an active ingredient, a compound according to formula I: 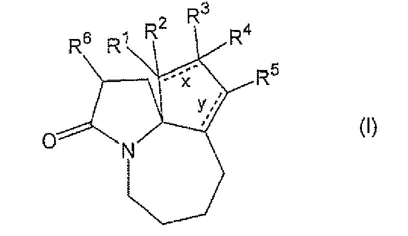 wherein
   R¹ and R² are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
   R¹ and R², together with the carbon atom to which they are attached, may form an optionally substituted 4-, 5-, or 6-membered ring or a carbonyl group (> C =O);
   R³ and R⁴ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
   R³ and R⁴ may together form =CH-(CH₂)ₙ-Ar, =CH-R^{a}Ar, =CH-(CH₂)ₙ-Het, =CH-R^{a}Het, or an oxo group (=O);
   R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
   a broken line indicates that a double bond may be present and, when a double bond represented by x is present, either R¹ or R² and either R³ or R⁴ are absent;
   n is independently 0, 1, or 2;
   m is independently 0, 1, or 2;
   R^{a} is independently C₁₋₆ alkylene, C₃₋₁₀ cycloalkylene, C₂₋₆ alkenylene, C₃₋₁₀ cycloalkenylene, or C₂₋₆ alkynylene;
   R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R^{a}C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)ₘR^{d};
   R^{d} is independently H, C₁₋₆ alkyl, C₂₋₆ alkenyl, C₂₋₆ alkynyl, C₃₋₁₀ cycloalkyl, or -Ar; R^{e} and R^{f} are independently H or C₁₋₆ alkyl;
   Ar is independently optionally substituted aryl; and
   Het is independently an optionally substituted 4-, 5-, or 6-membered heterocyclic group,
   or a salt, solvate or physiologically functional derivative thereof.
(7) The pharmaceutical composition according to (6),
   wherein
   R¹ is OH or H and R² is -R^{a}CO₂R^{d}, or
   R¹ and R², together with the carbon atom to which they are attached, for a carbonyl group (>C=O) or a 5-membered ring according to formula II: 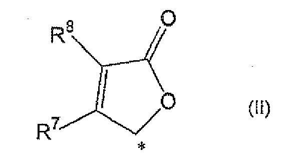 wherein
   R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
   * represents the carbon to which R¹ and R² are attached;
   R³ and R⁴ are both H, or
   R³ and R⁴ together form =CH-(CH₂)ₙ-Ar or an oxo group (=O),
   provided that, when a double bond represented by x is present, R¹ and R³ are absent;
   R⁵ is H or C₁₋₆ alkyl;
   R⁶ is H; and
   R^{a} to, R^{d}, n, and m are as defined in (6).
(8) The pharmaceutical composition according to (7),
   wherein
   R¹ is OH or H and R² is -C ≡ C-CO₂-C₁₋₆ alkyl, or
   R¹ and R², together with the carbon atom to which they are attached, form a carbonyl group (>C=O) or a 5-membered ring according to formula II: 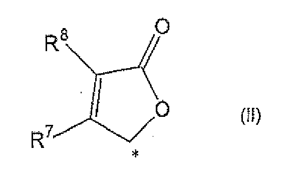 wherein
   R⁷ is C₁₋₆ alkoxy and R⁸ is C₁₋₆ alkyl or halogen;
   R³ and R⁴ are both H, or
   R³ and R⁴ together form =CH-phenyl, =CH-halophenyl, or an oxo group (=O), provided that, when a double bond represented by x is present, R¹ and R³ are absent;
   R⁵ is H or C₁₋₆ alkyl;
   R⁶ is H; and
   R^{a} to R^{d} and m are as defined in (6).
(9) The pharmaceutical composition according to (6), which comprises, as an active ingredient, a compound selected from the group consisting of the compounds below 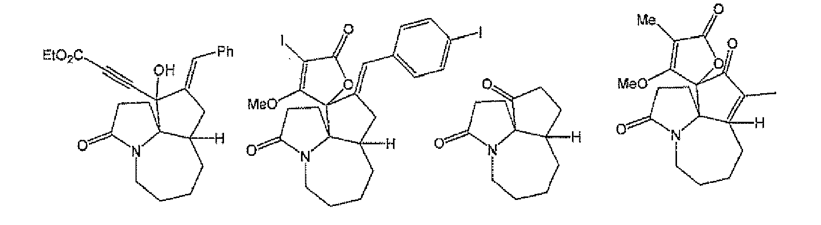 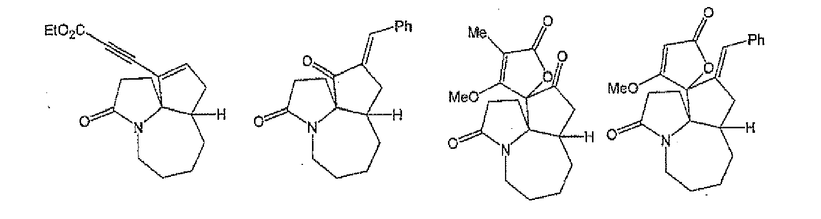 or a salt, solvate or physiologically functional derivative thereof.
(10) The pharmaceutical composition according to any of (6) to (9), wherein cancer is pancreatic cancer, gastric cancer, colon cancer, renal cancer, liver cancer, bone marrow cancer, adrenal cancer, skin cancer, melanoma, lung cancer, small intestinal cancer, prostate cancer, testicular cancer, uterine cancer, breast cancer, or ovarian cancer.
(11) The pharmaceutical composition according to (10), wherein cancer is pancreatic cancer.

The present invention provides a compound effective for prevention and/or treatment of cancer.

### Brief Description of the Drawings

Fig. 1 shows a chart showing the activity of the compound of the present invention for inhibiting Pim-3.
Fig. 2 shows a chart showing the activity of the compound of the present invention for inhibiting the growth of the PCI35 cell line.
Fig. 3 shows a chart showing the activity of the compound of the present invention for inhibiting the growth of the PCI55 cell line.
Fig. 4 shows a chart showing the activity of the compound of the present invention for inhibiting the growth of the PANC-1 cell line.
Fig. 5 shows a chart showing the activity of the compound of the present invention for inhibiting the growth of the L3.6p1 cell line.
Fig. 6 shows a chart showing the activity of the compound of the present invention for inhibiting the growth of the MiaPaCa-2 cell line.
Fig. 7 shows a chart showing the activity of the compound of the present invention for inhibiting the growth of the PCI66 cell line.
Fig. 8 shows a chart showing the activity of the compound of the present invention for inhibiting the growth of the human liver cancer cell line.
Fig. 9 shows a chart showing the activity of the compound of the present invention for inhibiting the growth of the human colon cancer cell line.

This description includes part or all of the contents as disclosed in the description and/or drawings of Japanese Patent Application No. 2008-160878, which is a priority document of the present application.

### Embodiments of the Invention

An aspect of the present invention relates to a compound according to formula I or a salt, solvate or physiologically functional derivative thereof: 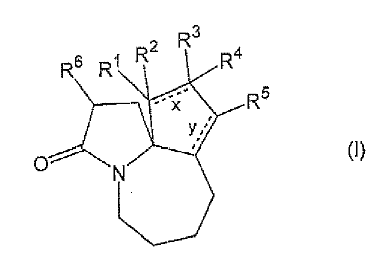 wherein
R¹ and R² are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R¹ and R², together with the carbon atom to which they are attached, may form an optionally substituted 4-, 5-, or 6-membered ring, preferably a heterocyclic ring, or a carbonyl group (>C=O);
R³ and R⁴ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R³ and R⁴ may together form =CH-(CH₂)ₙ-Ar, =CH-R^{a}Ar, =CH-(CH₂)ₙ-Het, =CH-R^{a}Het, or an oxo group (=O);
R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d} -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
a broken line indicates that a double bond may be present and, when a double bond represented by x is present, either R¹ or R² and either R³ or R⁴ are absent;
n is independently 0, 1, or 2;
m is independently 0, 1, or 2;
R^{a} is independently C₁₋₆ alkylene, C₃₋₁₀ cycloalkylene, C₂₋₆ alkenylene, C₃₋₁₀ cycloalkenylene, or C₂₋₆ alkynylene;
R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R^{a}C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)ₘR^{d};
R^{d} is independently H, C₁₋₆ alkyl, C₂₋₆ alkenyl, C₂₋₆ alkynyl, C₃₋₁₀ cycloalkyl, or -Ar; R^{e} and R^{f} are independently H or C₁₋₆ alkyl;
Ar is independently optionally substituted aryl; and
Het is independently an optionally substituted 4-, 5-, or 6-membered heterocyclic group,
except for a case in which R¹ and R², together with the carbon atom to which they are attached, form a 5-membered ring according to the following formula: 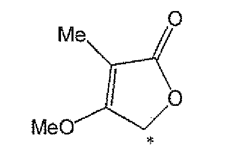 wherein * represents the carbon to which R¹ and R² are attached, R³ and R⁴ together form an oxo group (=O), R⁵ is a methyl group, a double bond represented by y is present, and R⁶ is H.
In the above compound, preferably, R¹ is OH or H, and R² is -R^{a}CO₂R^{d}, more preferably -C≡C-CO₂-C₁₋₆ alkyl, and particularly preferably -C≡C-CO₂-CH₂CH₃, or
R¹ and R², together with the carbon atom to which they are attached, form a carbonyl group (>C=O) or a 5-membered ring according to formula II: 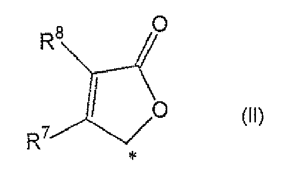 wherein
R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{C}, -R^{a}NR^{b}R^{C}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide,
preferably, R⁷ is C₁₋₆ alkoxy (methoxy in particular), and R⁸ is C₁₋₆ alkyl (methyl in particular), or halogen (iodine in particular), and
* represents the carbon to which R¹ and R² are attached.
In the above compound, preferably, R³ and R⁴ are both H, or R³ and R⁴ together form =CH-(CH₂)ₙ-Ar, and preferably =CH-Ar, or an oxo group (=O), in which Ar preferably represents optionally substituted phenyl, preferably phenyl or halophenyl, and particularly preferably iodophenyl, such as 4-iodophenyl.

In the above compound, when a double bond represented by x is present, R¹ and R³ are absent. When a double bond represented by x is present, accordingly, R¹ and R², together with the carbon atom to which they are attached, do not form an optionally substituted 4-, 5-, or 6-membered ring or a carbonyl group (>C=O), and R³ and R⁴ do not together form =CH-(CH₂)ₙ-Ar, =CH-R^{a}Ar, =CH-(CH₂)ₙ-Het, =CH-R^{a}Het, or an oxo group (=O).

In the above compound, R⁵ is preferably H or C₁₋₆ alkyl, such as methyl.

In the above compound, R⁶ is preferably H.

Specific examples of compounds according to formula I include the following compounds.

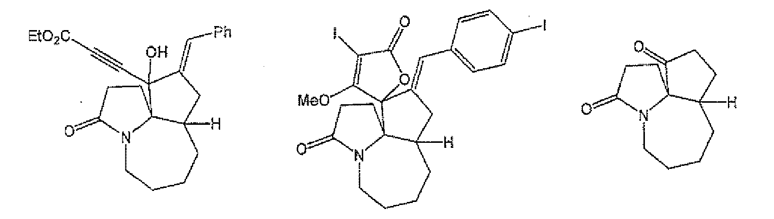

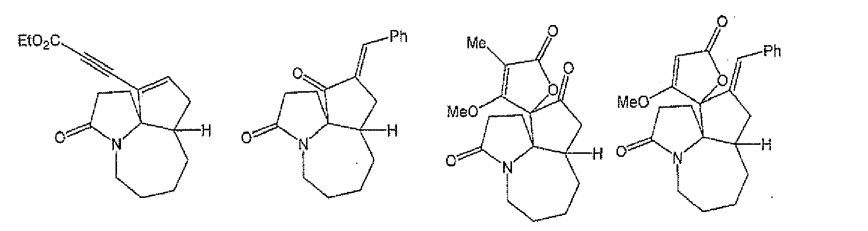

The compound according to formula I of the present invention can be synthesized via radical cyclization using Bu₃SnH and ACN [1,1'-azobis(cyclohexane-1-carbonitrile)] as catalysts.

The present invention also relates to a pharmaceutical composition comprising the compound according to formula I or a salt, solvate or physiologically functional derivative thereof. Preferably, the present invention relates to the pharmaceutical composition for prevention and/or treatment of cancer.

Another aspect of the present invention relates to a pharmaceutical composition for prevention and/or treatment of cancer comprising, as an active ingredient, a compound according to formula I or a salt, solvate or physiologically functional derivative thereof: 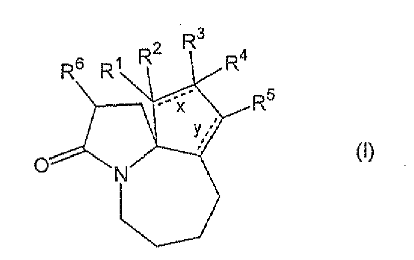 wherein
R¹ and R² are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R¹ and R², together with the carbon atom to which they are attached, may form an optionally substituted 4-, 5-, or 6-membered ring, preferably a heterocyclic ring, or a carbonyl group (>C=O);
R³ and R⁴ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R³ and R⁴ may together form =CH-(CH₂)ₙ-Ar, =CH-R^{a}Ar, =CH-(CH₂)ₙ-Het, =CH-R^{a}Het, or an oxo group (=O);
R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
a broken line indicates that a double bond may be present and, when a double bond represented by x is present, either R¹ or R² and either R³ or R⁴ are absent;
n is independently 0, 1, or 2;
m is independently 0, 1, or 2;
R^{a} is independently C₁₋₆ alkylene, C₃₋₁₀ cycloalkylene, C₂₋₆ alkenylene, C₃₋₁₀ cycloalkenylene, or C₂₋₆ alkynylene;
R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R^{a}C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)mR^{d};
R^{d} is independently H, C₁₋₆ alkyl, C₂₋₆ alkenyl, C₂₋₆ alkynyl, C₃₋₁₀ cycloalkyl, or -Ar;
R^{e} and R^{f} are independently H or C₁₋₆ alkyl;
Ar is independently optionally substituted aryl; and
Het is independently an optionally substituted 4-, 5-, or 6-membered heterocyclic group.
In the pharmaceutical composition for prevention and/or treatment of cancer, preferably,
R¹ is OH or H and R² is -R^{a}CO₂R^{d}, preferably -C≡C-CO₂-C₁₋₆ alkyl, and particularly preferably -C≡C-CO₂-CH₂CH₃, or
R¹ and R², together with the carbon atom to which they are attached, may form a carbonyl group (>C=O) or a 5-membered ring according to formula II: 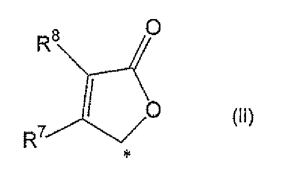 wherein
R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{C}, -R^{a}NR^{b}R^{C}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide,
preferably, R⁷ is C₁₋₆ alkoxy (methoxy in particular), and R⁸ is C₁₋₆ alkyl (methyl in particular), or halogen (iodine in particular); and
* represents the carbon to which R¹ and R² are attached.

In the above pharmaceutical composition for prevention and/or treatment of cancer, preferably,
R³ and R⁴ are both H, or
R³ and R⁴ together form =CH-(CH₂)ₙ-Ar, preferably =CH-Ar, or an oxo group (=O), and Ar is preferably optionally substituted phenyl, preferably phenyl or halophenyl, and particularly preferably iodophenyl, such as 4-iodophenyl.
In the pharmaceutical composition for prevention and/or treatment of cancer, when a double bond represented by x is present, R¹ and R³ are absent. When a double bond represented by x is present, accordingly, R¹ and R², together with the carbon atom to which they are attached, do not form an optionally substituted 4-, 5-, or 6-membered ring or a carbonyl group (>C=O), and R³ and R⁴ do not together form =CH-(CH₂)ₙ-Ar, -CH-R^{a}Ar, =CH-(CH₂)ₙ-Het, =CH-R^{a}Het, or an oxo group (=O).
In the pharmaceutical composition for prevention and/or treatment of cancer, R⁵ is preferably H or C₁₋₆ alkyl, such as methyl.
In the pharmaceutical composition for prevention and/or treatment of cancer, R⁶ is preferably H.

The pharmaceutical composition for prevention and/or treatment of cancer preferably comprises, as an active ingredient, a compound selected from the group consisting of the compounds below 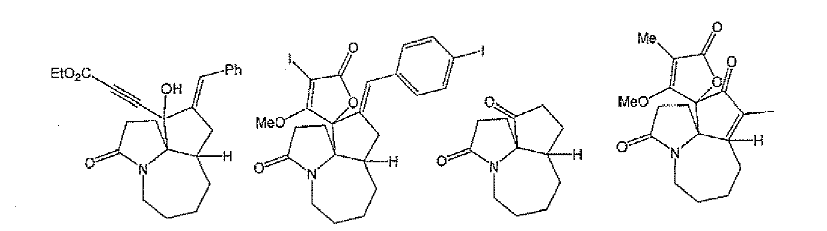 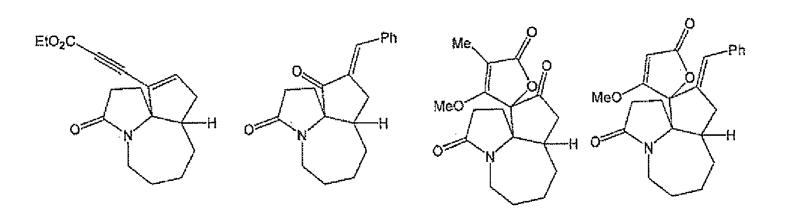 or a salt, solvate or physiologically functional derivative thereof.

The term "alkyl" used herein refers to a linear or branched saturated hydrocarbon group. Examples of "alkyl" include, but are not limited to, methyl, ethyl, propyl, isopropyl, isobutyl, n-butyl, tert-butyl, isopentyl, and n-pentyl.

The number of atoms (e.g., carbon atoms) is indicated as, for example, "C_{x-y} alkyl," and the term "C₁₋₆ alkyl" refers to alkyl having 1 to 6 carbon atoms.

The term "alkenyl" used herein refers to a linear or branched aliphatic hydrocarbon group having at least one carbon-carbon double bond. Examples thereof include, but are not limited to, vinyl and allyl.

The term "alkynyl" used herein refers to a linear or branched aliphatic hydrocarbon group having at least one carbon-carbon triple bond. An example thereof is, but is not limited to, ethynyl.

The term "alkylene" used herein refers to a linear or branched divalent saturated hydrocarbon group. Examples of "alkylene" include, but are not limited to, methylene, ethylene, n-propylene, and n-butylene.

The term "alkenylene" used herein refers to a linear or branched divalent hydrocarbon group having at least one carbon-carbon double bond. Examples thereof include, but are not limited to, vinylene, allylene, and 2-propylene.

The term "alkynylene" used herein refers to a linear or branched divalent hydrocarbon group having at least one carbon-carbon triple bond. An example thereof is, but is not limited to, ethynylene.

The term "cycloalkyl" used herein refers to a substituted or unsubstituted non-aromatic hydrocarbon cyclic group. Examples of "cycloalkyl," include, but are not limited to, cyclopropyl, cyclobutyl, cyclopentyl, cyclohexyl, and cycloheptyl. Examples of preferable substituents include C₁₋₆ alkyl, C₂₋₆ alkenyl, C₂₋₆ alkynyl, C₁₋₆ alkoxy, hydroxyl, oxo, halogen, C₁₋₆ haloalkyl, C₃₋₁₀ cycloalkyl, C₃₋₁₀ cycloalkoxy, cyano, amide, amino, and C₁₋₆ alkylamino groups.

The term "cycloalkenyl" used herein refers to a substituted or unsubstituted nonaromatic hydrocarbon cyclic group having or free of an alkylene linker and having at least one carbon-carbon double bond. Examples of "cycloalkenyl" include, but are not limited to, cyclopropenyl, cyclobutenyl, cyclopentenyl, cyclohexenyl, and cycloheptenyl. Examples of preferable substituents of nonaromatic hydrocarbon cyclic groups include C₁₋₆ alkyl, C₂₋₆ alkenyl, alkynyl, C₁₋₆ alkoxy, hydroxyl, oxo, halogen, C₁₋₆ haloalkyl, C₃₋₁₀ cycloalkyl, C₃₋₁₀ cycloalkoxy, cyano, amide, amino, and C₁₋₆ alkylamino groups.

The term "cycloalkylene" used herein refers to a substituted or unsubstituted divalent nonaromatic hydrocarbon cyclic group. Examples of "cycloalkylene" include, but are not limited to, cyclopropylene, cyclopropylene, cyclobutylene, cyclopentylene, cyclohexylene, and cycloheptylene.

The term "cycloalkenylene" used herein refers to a substituted or unsubstituted divalent nonaromatic hydrocarbon cyclic group having at least one carbon-carbon double bond. Examples of "cycloalkenylene" include, but are not limited to, cyclopropenylene, cyclobutenylene, cyclopentenylene, cyclohexenylene, and cycloheptenylene.

The term "heterocyclic group" used herein refers to a monocyclic or polycyclic system containing at least one hetero atom. Examples thereof include an aromatic heterocyclic group and a nonaromatic heterocyclic group. Examples of preferable hetero atoms include N, O, and S, and examples further include N-oxide, sulfur oxide, and dioxide.

A "nonaromatic heterocyclic group" is saturated or has a degree of unsaturation of 1 or higher, and it is preferably a monocyclic group. Examples of nonaromatic heterocyclic groups include, but are not limited to, tetrahydrofuranyl, pyranyl, 1,4-dioxanyl, 1,3- dioxanyl, piperidinyl, piperazinyl, pyrrolidinyl, morpholinyl, azetidinyl, tetrahydrothiopyranyl, and tetrahydrothiophenyl. Examples of preferable substituents include C₁₋₆ alkyl, C₂₋₆ alkenyl, C₂₋₆ alkynyl, C₁₋₆ alkoxy, hydroxyl, C₁₋₆ alkyl hydroxy, halogen, C₁₋₆ haloalkyl, C₃₋₁₀ cycloalkyl, C₃₋₁₀ cycloalkoxy, cyano, amide, amino, C₁₋₆ alkylamino, and imidamide groups (i.e., -C(NH)NH₂ and substitution groups thereof).

An "aromatic heterocyclic group" is preferably a monocyclic group. Examples of aromatic heterocyclic group include, but are not limited to, furanyl, thiophenyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyridazinyl, pyrazinyl, pyrimidinyl, quinolinyl, isoquinolinyl, benzofuranyl, benzothiophenyl, indolyl, indazoyl, benzoimidazolyl, imidazopyridinyl, pyrazolopyridinyl, and pyrazolopyrimidinyl. Examples of preferable substituents include C₁₋₆ alkyl, C₂₋₆ alkenyl, C₂₋₆ alkynyl, C₁₋₆ alkoxy, hydroxyl, halogen, C₁₋₆ haloalkyl, C₃₋₁₀ cycloalkyl, C₃₋₁₀ cycloalkoxy, cyano, amide, amino, and C₁₋₆ alkylamino groups.

The term "aryl" used herein refers to a benzene ring or condensed benzene ring system, such as an anthracene, phenanthrene, or naphthalene ring system. Examples of "aryl" groups include, but are not limited to, phenyl, 2-naphthyl, and 1-naphthyl. Examples of preferable substituents include C₁₋₆ alkyl, C₂₋₆ alkenyl., C₂₋₆ alkynyl, C₁₋₆ alkoxy, hydroxyl, halogen, C₁₋₆ haloalkyl, C₃₋₁₀ cycloalkyl, C₃₋₁₀ cycloalkoxy, cyano, amide, amino, and C₁₋₆ alkylamino groups.

The term "halogen" used herein refers to fluorine, chlorine, bromine, or iodine.

The term "haloalkyl," refers to an alkyl group substituted with at least one halogen. Examples of "halo alkyl groups include, but are not limited to, methyl, ethyl, propyl, isopropyl, n-butyl, and t-butyl independently substituted with at least one halogen, such as a fluoro, chloro, bromo, or iodo group.

The term "halophenyl" used herein refers to a phenyl group substituted with at least one halogen.

The term "alkoxy" used herein refers to an -OR' group, wherein R' is alkyl. The term "cycloalkoxy" used herein refers to an -OR' group, wherein R' is cycloalkyl.

The term "amino" used herein refers to an -NR'R" group, wherein R' and R" are independently H, C₁₋₆ alkyl, C₂₋₆ alkenyl, C₂₋₆ alkynyl, C₃₋₆ cycloalkyl, aryl, or a heterocyclic group.

The term "amide" used herein refers to a -C(O)NR'R" group, wherein R' and R" are independently H, C₁₋₆ alkyl, C₂₋₆ alkenyl, C₂₋₆ alkynyl, C₃₋₆ cycloalkyl, aryl, or a heterocyclic group. Examples of "amide" include -C(O)NH₂, -C(O)NH(CH₃), and -C(O)N(CH₃)₂ groups.

The compound of the present invention is sometimes crystallized in one or more forms (known as a polymorphism), and such polymorphic forms are within the scope of the present invention. In general, a polymorphism results from changes in temperature, pressure, or both thereof. A polymorphism can be identified based on physical properties known in the art, such as X-ray diffraction patterns, solubility, or melting points.

The compound of the present invention has one or more chiral center or may be present as various stereoisomers. Mixtures of isomers (e.g., stereoisomers, positional isomers, geometric isomers, or optical isomers) and purified isomers are within the scope of the present invention.

A salt of the compound of the present invention is a pharmaceutically acceptable salt, in general. The term "pharmaceutically acceptable salt" refers to a nontoxic salt of the compound of the present invention. An example of a salt of the compound of the present invention is an acid addition salt. Examples of representative salts include acetate, benzene sulfonate, benzoate, carbonate, sulfate, tartarate, borate, calcium edetate, camsilatea, clavulanate, citrate, edisylate, fumarate, gluconate, glutamate, hydrobromate, hydrochloride, hydroxy naphthoic acid salt, isothionate, lactate, lactobionate, laurate, malate, maleate, mandelate, mesylate, methylsulfate, monopotassium maleate, mucate, napsylate, nitrate, oxalate, palmitate, pantothenate, phosphate/diphosphate, potassium salt, salicylate, sodium salt, stearate, subacetate, succinate, tannate, tosylate, triethiodide, trimethylammonium salt, and valerate.

The term "solvate" used herein refers to a complex composed of a solute (the compound according to formula 1, or a salt or physiologically functional derivative thereof) and a solvent. Examples of solvent include, but are not limited to, water, methanol, ethanol, and acetic acid.

The term "physiologically functional derivative" used herein refers to every type of pharmaceutically acceptable derivative of the compound of the present invention that is capable of providing the compound of the present invention or an active metabolic product thereof (directly or indirectly) at the time of administration to mammals. A person skilled in the art would readily conceive of such derivative, such as an ester and amide, without undue experimentation (Burger's Medicinal Chemistry And Drug Discovery, 5th edition, vol 1: Principles and Practice).

The compound according to formula I of the present invention or a salt, solvate or physiologically functional derivative thereof is effective for prevention and/or treatment of cancer. Administration of an effective amount of the compound according to formula I of the present invention or a salt, solvate or physiologically functional derivative thereof to a patient who is in need thereof enables prevention and/or treatment of cancer.

The term "effective amount" refers to the amount of a compound that induces biological or medical responses in a tissue, system, animal, or human as desired by researchers or clinicians, for example. An effective amount of the compound of the present invention varies depending on, for example, the age, body weight, severity of a patient, properties of a pharmaceutical preparation, and the route of administration. An effective amount of the compound according to formula I for treating mammals, particulary humans, is generally 0.1 to 100 mg/kg/day, such as from 0.1 to 10 mglkg/day.

In the present invention, cancers include pancreatic cancer, gastric cancer, colon cancer, renal cancer, liver cancer, bone marrow cancer, adrenal cancer, skin cancer, melanoma, lung cancer, small intestinal cancer, prostate cancer, testicular cancer, uterine cancer, breast cancer, and ovarian cancer. The pharmaceutical composition for prevention and/or treatment of cancer of the present invention is particularly effective for prevention and/or treatment of pancreatic cancer. Since the compound according to formula I of the present invention has effects of inhibiting Pim-3 activity, the pharmaceutical composition for prevention and/or treatment of cancer of the present invention is particularly effective on cancer in which elevated Pim-3 expression is observed. The compound according to formula I of the present invention can be used as an inhibitor of Pim-3 activity.

In the present invention, disease prevention includes suppression and retardation of the onset of a disease. It includes not only prevention at the preclinical stage but also prevention of relapse after treatment. In the present invention, treatment of a disease includes the curing of a disease, symptomatic relief, and suppression of symptom progression.

The target of administration of the pharmaceutical composition of the present invention is preferably a mammal. The term "mammal" used herein refers to a warm-blooded vertebrate. Examples include primates such as humans and monkeys, rodents such as mice, rats, and rabbits, pet animals such as dogs and cats, and livestock animals such as cattles, horses, and pigs. The composition of the present invention is preferable for administration to primates, and to humans in particular.

The pharmaceutical composition of the present invention comprises the compound according to formula I or a salt, solvate or physiologically functional derivative thereof and at least one pharmaceutically acceptable carrier. The term "pharmaceutically acceptable carrier" generally refers to an inactive, nontoxic, and solid or liquid extender, diluent, or encapsulated material that does not react with the active ingredient of the present invention. Examples thereof include water, ethanol, polyol (e.g., glycerol, propylene glycol, and liquid polyethylene glycol), an adequate mixture thereof, a solvent and a dispersion medium such as vegetable oil.

The pharmaceutical composition of the present invention is administered orally or parenterally. Examples of administration routes include cutaneous, hypodermic, mucosal, intravenous, intraarterial, intramuscular, intraperitoneal, vaginal, intrapulmonary, intracerebral, intraocular, and intranasal routes. Examples of forms of pharmaceutical preparations for oral administration include tablets, granules, subtle granules, powders, capsules, chewable agents, pellets, syrup, liquid drugs, suspensions, and inhalants. Examples of forms of pharmaceutical preparations for parenteral administration include suppositories, retention enemas, drops, eye drops, nasal drops, pessaries, injection solutions, mouthwashes, and external agents for application to the skin, such as ointments, creams, gels, controlled-release patches, and adhesive skin patches. The pharmaceutical composition of the present invention may be administered parenterally in the form of sustained-release subcutaneous implants or a targeted drug delivery system (e.g., a monoclonal antibody, vector delivery, ion implantation, polymer matrix, liposome, or microsphere).

The pharmaceutical composition of the present invention may further comprise additives that are common in the field of medicine. Examples of such additives include excipients, binders, disintegrators, lubricants, antioxidants, colorants, and flavors, and such additives can be used according to need. In order to prepare long-acting sustained-release agents, coating with the use of retardants or the like can be carried out. Examples of excipients include carboxymethylcellulose sodium, agar, light anhydrous silicic acid, gelatin, crystalline cellulose, sorbitol, talc, dextrin, starch, lactose, saccharose, glucose, mannitol, magnesium aluminometasilicate, and calcium hydrogen phosphate. Examples of blinders include gum arabic, sodium alginate, ethanol, ethyl cellulose, sodium caseinate, carboxymethylcellulose sodium, agar, purified water, gelatin, starch, gum tragacanth, lactose, hydroxycellulose, hydroxymethylcellulose, hydroxypropylcellulose, and polyvinyl pyrrolidone. Examples of disintegrators include carboxymethylcellulose, carboxymethylcellulose sodium, carboxymethylcellulose calcium, crystalline cellulose, starch, and hydroxypropyl starch. Examples of lubricants include stearic acid, calcium stearate, magnesium stearate, talc, hydrogenated oil, sucrose fatty acid ester, and waxes. Examples of antioxidants include tocopherol, gallic acid ester, dibutyl hydroxytoluene (BHT), butylated hydroxyanisole (BHA), and ascorbic acid. Other additives or drugs, such as antacids (e.g., sodium bicarbonate, magnesium carbonate, precipitated calcium carbonate, or hydrotalcite) or gastric coating agents (e.g., synthetic aluminum silicate, sucralfate, or sodium copper chlorophyllin) may be added according to need.

### EXAMPLES

### (Example 1)

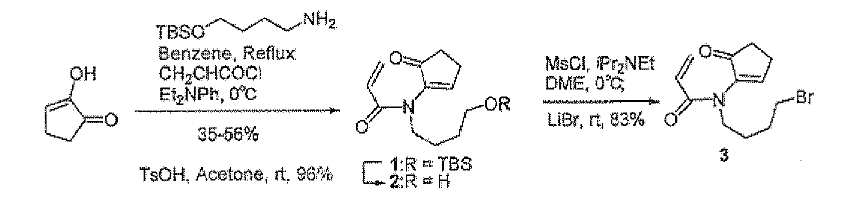

1,2-Cyclopentanedione and 4-(t-butyldimethylsiloxy)butylamine were condensed, and the resulting imine was acylated with acryloyl chloride in the presence of N,N-diethylaniline to obtain enamide (Compound 1). A TBS group was removed from Compound 1, alcohol (Compound 2) was mesylated, and the resultant was brominated with lithium bromide to obtain Compound 3.

### (Example 2)

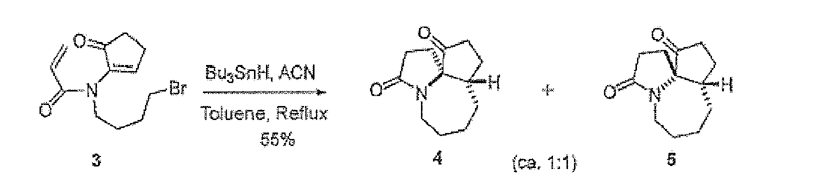

A solution of Bu₃SnH (1.53 g, 5.24 mmol) and ACN [1,1-azobis(cyclohexane-1-carbonitrile)] (171 mg, 0.699 mmol) in toluene (100 ml) was added dropwise to a solution of Compound 3 (1.00 g, 3.50 mmol) in reflux toluene (350 ml) over a period of 5 hours, and the reaction solution was heated to reflux for an additional 1 hour. After the solvent was removed by distillation, the residue was purified via silica gel column chromatography containing 10% (w/w) potassium fluoride, and a mixture of Compound 4 and Compound 5 was then obtained (399 mg, 55%). This mixture was recrystallized, and Compound 5 alone was obtained.

### Compound 4 and Compound 5:

IR (CHCl₃) υ 1680, 1745 cm⁻¹; ¹H NMR (270 MHz, CDCl₃) δ 1.31-1.43 (1H, m), 1.50-2.07 (9 H, m), 2.15-2.06 (6 H, m), 4.04 (1/2 H, d, J = 14.6 Hz), 4.12 (1/2 H, d, J = 14.6 Hz); ¹³C NMR (67.8 MHz, CDCl₃) δ 22.6, 23.1, 23.9, 24.2, 25.5, 27.58, 27.63, 28.0, 28.6, 29.4, 30.1, 30.7, 34.3, 34.7, 39.6, 40.3, 44.6, 46.9, 73.3, 74.2,175.4, 176.0, 212.4. Anal. Calcd for C₁₂H₁₇NO₂: C, 69.54; H, 8.27; N, 6.76. Found: C, 69.34; H, 8.50; N, 6.88.

### (Example 3)

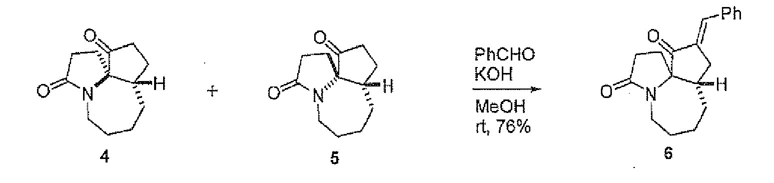

A mixture of Compound 4 and Compound 5 (1:1, 399 mg, 1.93 mmol) was dissolved in 7 ml of 10% KOH methanol, benzaldehyde (225 mg, 2.12 mmol) was added thereto, and the mixture was agitated for 24 hours. An aqueous saturated ammonium chloride solution was added to the reaction mixture, followed by extraction with ethyl acetate. The organic layer was washed with saturated saline and dried over magnesium sulfate. The solvent was removed by distillation, the residue was purified via silica gel column chromatography, and Compound 6 (433 g, 76%) was obtained in the form af a stereoisomer mixture (1:1).

IR (CHCl₃) υ 1628, 1682, 1721 cm-¹; ¹H NMR (500 MHz, CDCl₃) δ 1.39-1.97 (8 H, m), 2.03-2.13 (1H, m), 2.25-2.34 (1H, m), 2.45-2.68 [(2 + 1/2) H, m], 2.73 (1/2 H, td, J = 14.6, 3.7 Hz), 3.00 (1/2 H, dd, J = 15.9, 6.7 Hz), 3.12 (1/2 H, ddd, J = 17.7, 8.5, 3.1 Hz), 4.04-4.07 (1/2 H, m), 4.20 (1/2 H, td, J = 10.4, 4.3 Hz), 7.27-7.58 (6 H, m); ¹³C NMR (125 MHz, CDCl₃) δ 22.9, 25.1, 25.3, 26.6, 27.7, 28.9, 29.4, 29.5, 30.0, 30.4, 30.8, 32.0, 39.47, 39.51, 42.8, 44.8, 73.9, 74.3, 128.78, 128.84, 129.8, 130.0, 130.4, 130.9,132.1, 132.9, 134.6, 134.8, 134.9, 136.0, 175.4, 176.3, 202.3, 206.3; HRMS calcd for C₁₉H₂₁NO₂ 295.1572, found 295.1572.

### (Example 4)

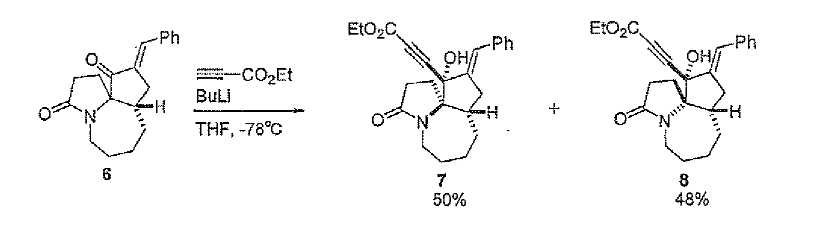

A 1.6M hexane solution of butyllithium (1.46 ml, 2.34 mmol)) was added to 5 ml of a THF solution of ethyl propiolate (230 g, 2.34 mmol) at -78°C, and the mixture was agitated at that temperature for 30 minutes. A THF solution (5 ml) of Compound 6 (230 mg, 0.788 mmol) was added thereto, and the mixture was agitated at that temperature for 20 minutes. An aqueous saturated ammonium chloride solution was added to the reaction mixture, followed by extraction with ethyl acetate. The organic layer was washed with saturated saline and dried over magnesium sulfate. The solvent was removed by distillation, the residue was purified via silica gel column chromatography, Compound 7 (151 mg, 50%) was obtained from the first fraction, and Compound 8 (149 mg, 48%) was obtained from the second fraction.

Compound 7: mp 209-211°C (EtOAc-MeOH): IR (CHCl₃) υ 1705, 1675 cm⁻¹; ¹H NMR (270 MHz, CDCl₃) δ 1.29 (3H, t, J = 7.1 Hz), 1.40-1.50 (3H, m), 1.72-1.84 (4H, m), 2.06-2.40 (3H, m), 2.67-2.92 (3H, m), 3.24 (1H, dd, J = 14.3, 10.2 Hz), 4.17-4.26 (1H, m), 4.22 (2H, q, J = 7.1 Hz), 5.87 (1H, br), 6.87 (1H, t-like), 7.21-7.40 (5H, m); ¹³C NMR (67.5 MHz, CDCl₃) δ 14.0, 24.0,25.7, 28.4, 30.3, 30.5, 31.7, 41.2, 42.2, 62.0, 76.2, 77.6, 79.3, 87.5, 127.2, 127.3, 128.4, 128.7, 136.8, 140.5, 153.2, 178.3. Anal. Calcd for C₂₄H₂₇NO₄: C, 73.26; H, 6.92; N, 3.56. Found: C, 73.29; H, 7.00; N, 3.55.

Compound 8: mp. 190.5-192°C (EtOAc-MeOH): IR (CHCl₃) υ 1705, 1675 cm⁻¹; ¹H NMR (270 MHz, CDCl₃) δ 1.21-1.35 (1H, m), 1.30 (3H, t, J = 7.1 Hz), 1.55-1.70 (4H, m), 1.83-2.06 (2H, m), 2.23-2.32 (2H, m), 2.62-2.92 (5H, m), 3.90 (1H, d, J = 14.3 Hz), 4.01 (1H, br), 4.23 (2H, q, J = 7.1 Hz), 6.79 (1H, t-like), 7.26-7.31 (1H, m), 7.37-7.39 (4H, m); ¹³C NMR (67.5 MHz, CDCl₃) δ 14.0, 22.1, 25.1, 28.2, 28.4, 29.4, 30.3, 41.4, 62.2, 76.4, 79.0, 81.0, 86.7, 123.6, 123.7, 127.4, 128.5, 129.0, 1.36.2, 142.1, 153.3, 176.3. Anal. Calcd for C₂₄H₂₇NO₄: C, 73.26; H, 6.92; N, 3.56. Found: C, 73.30; H, 6.99; N, 3.57.

### (Example 5)

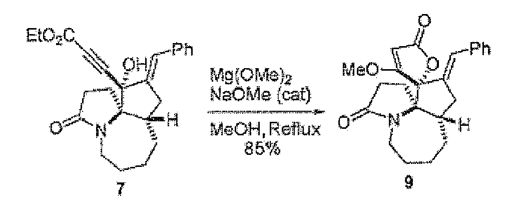

A methanol solution (1.5 ml) containing 6% to 10% magnesium methoxide was added to 2 ml of a methanol solution of Compound 7 (159 mg, 0.4 mmol), and the mixture was heated to reflux for 10 hours. Further, sodium methoxide (4.3 mg, 0.08 mmol) was added, and the mixture was heated to reflux for 2 days. An aqueous saturated ammonium chloride solution was added to the reaction mixture, followed by extraction with ethyl acetate. The organic layer was washed with saturated saline and dried over magnesium sulfate. The solvent was removed by distillation, the residue was purified via silica gel column chromatography, and Compound 9 (129 mg, 85%) was obtained.

mp 247-248°C (EtOAc-CH₂Cl₂): IR (CHCl₃) υ 1632, 1682, 1761 cm⁻¹; ¹H NMR (500 MHz, CDCl₃) δ 1.35-1.41 (2 H, m), 1.54 (1H, m), 1.71-1.80 (3 H, m), 1.96 (1H, q, J = 11.6 Hz), 2.20-2.27 (2 H, m), 2.49 (1H, t, J = 13.4 Hz), 2.69 (1H, t, J = 12.2 Hz), 2.80-2.99 (3 H, m), 3.87 (3 H, s), 4.07 (1H, d, J = 14.0 Hz), 5.12 (1H, s), 6.37 (1H, s), 7.35-7.39 (5 H, m); ¹³C NMR (125 MHz, CDCl₃) δ 25.2, 26.1, 28.2, 30.0, 30.1, 32.2, 40.1, 41.2, 59.6, 75.0, 89.3, 94.4, 127.8, 127.9, 128.5, 128.7, 135.6, 135.8, 171.0, 177.9, 181.5; HRMS calcd for C₂₃H₂₅NO₄, 379.1784, found 379.1772.

### (Example 6)

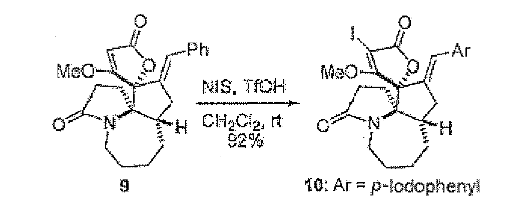

Trifluoromethanesulfonic acid (277 mg, 1.85 mmol) was added dropwise to a solution of Compound 9 (200 mg, 0.527 mmol) and N-iodosuccinimide (356 mg, 1.581 mmol) in CH₂Cl₂ (7 ml) under ice cooling, and the mixture was agitated at room temperature for 16 hours. CH₂Cl₂ was added to the reaction mixture, and the resultant was washed with an aqueous saturated sodium thiosulfate solution and with saturated saline. After the resultant was dried over magnesium sulfate, the solvent was removed by distillation, the residue was purified via silica gel column chromatography, and Compound 10 (306 mg, 92%) was obtained.

mp 234-237°C (dec) (EtOAc-CH₂Cl₂): IR (CHCl₃) υ 1617, 1686, 1757 cm⁻¹; ¹H NMR (270 MHz, CDCl₃) δ 1.33-1.58 (3 H, m), 1.73-1.83 (3 H, m), 1.89-2.05 (1H, m), 2.13-2.29 (2 H, m), 2.44 (1H, ddd, J = 16.2, 13.2, 3.0 Hz), 2.66-2.87 (3 H, m), 2.92-3.03 (1H, m), 4.11 (1H, d, J = 13.4 Hz), 4.42 (3 H, s), 6.28 (1H, t-like), 7.09 (2 H, d, J = 8.4 Hz), 7.69 (2 H, d, J = 8.4 Hz); ¹³C NMR (67.5 MHz, CDCl₃) δ 25.1, 26.1, 28.2, 29.8, 30.0, 32.1, 40.3, 41.2, 47.2, 60.3, 75.3, 93.6, 96.6, 127.3, 130.4, 135.0, 136.9, 137.6, 169.5, 177.9, 178.1; HRMS calcd for C₂₃H₂₃NO₄: I₂630.9717, found 630.9716.

### (Example 7)

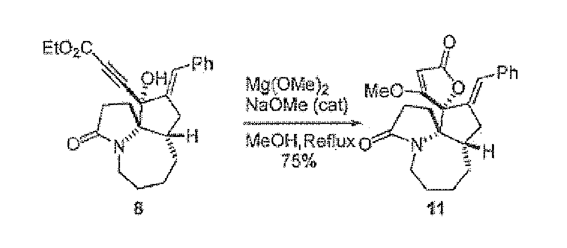

Compound 11 was synthesized from Compound 8 in the same manner as in the case of synthesis of Compound 9.

mp 239-246°C (dec) (EtOAc-CH₂Cl₂): IR (CHCl₃) υ 1632, 1678, 1765 cm⁻¹; ¹H NMR (500 MHz, CDCl₃) δ 1.24-1.28 (1 H, m), 1.57-1.67 (4 H, m), 1.88-1.97 (3 H, m), 2.24-2.3 (2 H, m), 2.39 (1 H, t, J = 13.4 Hz), 2.81-2.92 (2 H, m), 3.03 (1 H, d, J = 17.7, 5.5 Hz), 3.92 (3 H, s), 3.97 (1 H, d, J = 14.6 Hz), 5.00 (1 H, s), 6.49 (1 H, s), 7.28-7.30 (1 H, m), 7.37-7.40 (4 H, m); ¹³C NMR (125 MHz, CDCl₃) δ 22.0, 25.2, 28.1, 29.0, 29.3, 30.0, 40.9, 42.9, 60.0, 74.2, 85.9, 93.9, 124.1, 127.7, 128.6, 129.1, 135.8, 137.1, 171.4, 174.2, 184.3; HARMS calcd for C₂₃H₂₅NO₄: 379.1784, found 379.1790.

### (Example 8)

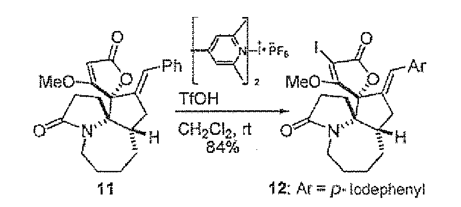

Trifluoromethanesulfonic acid (139 mg, 0.924 mmol) was added dropwise to a solution of Compound 11 (100 mg, 0.264 mmol) and bis(trimethylpyridine)iodonium hexafluorophosphate (488 mg, 0.949 mmol) in CH₂Cl₂ (10 ml) under ice cooling, and the mixture was agitated at room temperature for 24 hours. CH₂Cl₂ was added to the reaction mixture, and the resultant was washed with an aqueous saturated sodium thiosulfate solution and with saturated saline. After the resultant was dried over magnesium sulfate, the solvent was removed by distillation, the residue was purified via silica gel column chromatography, and Compound 12 (139 mg, 84%) was obtained.

mp 205-208°C (dec) (EtOAc-CH₂Cl₂): IR (CHCl₃) υ 1619, 1682, 1771 cm⁻¹; ¹H NMR (270 MHz, CDCl₃) δ 1.26-1.35 (1 H, m), 1.51-1.68 (4 H, m), 1.84-1.97 (3 H, m), 2.24-2.47 (3 H, m), 2.74-3.05 (3 H, m), 3.95 (1 H, d, J = 14.7 Hz), 4.44 (3 H, s), 6.40 (1 H, t-like), 7.10 (2 H, d, J = 8.4 Hz), 7.72 (2 H, d, J = 8.4 Hz); ¹³C NMR (67.5 MHz, CDCl₃) δ 22.0, 25.1, 28.0, 28.9, 29.4, 29.9, 40.9, 43.0, 43.9, 60.4, 74.7, 93.6, 96.2, 123.7, 130.7, 135.0, 137.7, 138.3, 169.7, 174.0, 180.6; HRMS calcd for C₂₃H₂₃NO₄: I₂630.9717, found 630.9728.

### (Example 9)

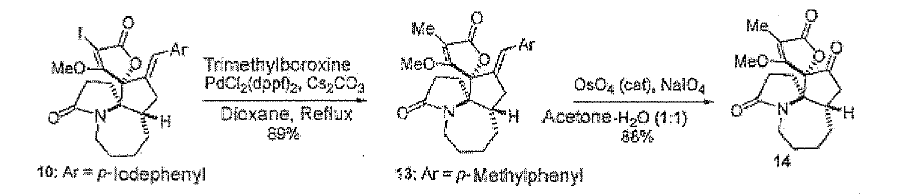

Five drops of an aqueous solution of 4% osmium oxide were added to a mixed solvent of Compound 13 (100 mg, 0.245 mmol) synthesized from Compound 10 and sodium periodate (2.60 g, 12.3 mmol) in acetone (10 ml) and water (10 ml), and the mixture was agitated at room temperature for 30 hours. Water was added to the reaction mixture, followed by extraction with CH₂Cl₂. After the organic layer was dried over magnesium sulfate, the solvent was removed by distillation, the residue was purified via silica gel column chromatography, and Compound 14 (68.5 mg, 88%) was obtained.

mp 260-269°C (dec) (EtOAc-CH₂Cl₂): IR (CHCl₃) υ 1619, 1686, 1781 cm⁻¹; ¹H NMR (270 MHz, CDCl₃) δ 1.24-1.67 (3 H, m), 1.78-1.85 (3 H, m), 2.00-2.12 (2 H, m), 2.03 (3 H, s), 2.21-2.37 (2 H, m), 2.60 (1 H, dd, J = 18.6, 7.7 Hz), 2.68-2.90 (2 H, m), 2.76 (1 H, dt, J = 8.4, 3.1 Hz), 4.11 (3 H, s), 4.14 (1. H, d, 3 = 12.0 Hz); ¹³C NMR (67.5 MHz, CDCl₃,) δ 9.1, 24.7, 26.0, 27.9, 29.6, 30.0, 38.0, 40.0, 40.3, 59.6, 73.5, 91.4, 100.1, 167.9, 177.4, 206.0; HRMS calcd for C₁₇H₂₁NO₅: 319.1420, found 319.1416.

### (Example 10)

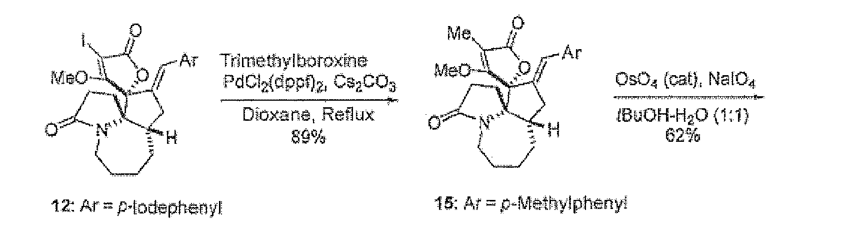

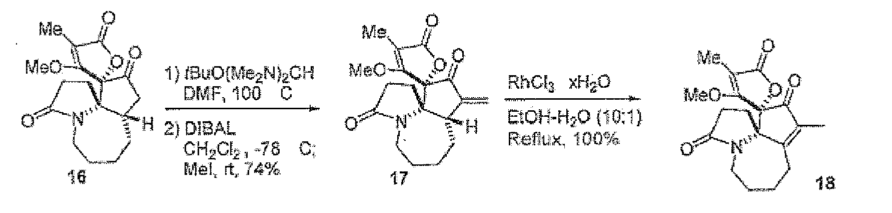

Rhodium (iii) chloride trihydrate (0.4 mg, 1.81 µmol) was added to a solution of Compound 17 (3.0 mg, 9.05 µmol) obtained through 3 steps from Compound 12 in ethanol and water (10:1) (0.5 ml), and the mixture was heated to reflux for 30 minutes. The solvent was removed by distillation, the residue was purified via silica gel column chromatography, and Compound 18 (3.0 mg, 100%) was obtained. The ¹H and ¹³C NMR spectral data for this compound were very consistent with those for isostemonamide.

mp 223-224°C (EtOAc-CH₂Cl₂): IR (CHCl₃) υ 1644, 1663, 1.690, 1721, 1763 cm⁻¹; ¹H NMR (500 MHz, CDCl₃) δ 1.25-1.45 (2 H, m), 1.78 (1 H, dd, J = 14.5, 3.7 Hz), 1.86 (3 H, s), 1.92 (1 H, td, J = 13.2, 9.3 Hz), 2.07 (3 H, s), 2.10-2.15 (2 H, m), 2.27 (1 H, ddd, J = 16.6, 12.2, 7.6 Hz), 2.35 (1 H, dd, J = 16.6, 9.3 Hz), 2.61 (1 H, dd, J = 13.4, 7.3 Hz), 2.95 (1 H, dd, J = 12.7, 6.6 Hz), 3.00 (1 H, t, J = 19.7 Hz), 4.15 (3 H, s), 4.17 (1 H, d, J = 15.0 Hz); ¹³C NMR (125 MHz, CDCl₃) δ 8.3, 9.3, 26.9, 27.7, 28.0, 29.4, 29.8, 42.4, 59.9, 73.5, 86.5, 102.9, 136.6, 168.7, 171.7, 172.6, 174.6, 196.9; HRMS calcd for C₁₈H₂₁NO₅: 331.1420, found 331.1417.

### (Example 11) In vitro assay

A His-Bad protein solution (3 µg/ml) diluted with a carbonate buffer (pH 9.6) (15 µmol/l Na₂CO₃ (Wako), 35 µmol/L NaHCO₃ (Wako), 0.02% (w/v) NaN₃ (Wako), pH 9.6) was added at 100 µl/well to a 96-well microplate (Nunc-Immuno^{™} Plate, NUNC Brand Products, Nalge Nunc International, Denmark), and the plate was coated at 4°C overnight. The plate was washed 3 times on the following day, a 1% BSA/PBS(-) solution was added at 150 µl/well, the plate was blocked at 37°C for 1 hour, and the plate was then washed 3 times. A kinase buffer containing 19.2 nmol/l thioredoxin-hexahistidine-Pim-3 fusion protein, 10 µmol/l ATP (Cell Signaling), and Compounds 5, 7, and 12 synthesized in the examples above (0, 20, 40, 80, and 160 µmol/l) (25 mmol/l Tris-HCl (pH 7.5), 10 mmol/l MgCl₂, 0.1 mmol/l Na₃VO₄, 2 mmol/l dithiothreitol (DTT), 5 mmol/l s-glycerophosphate, Cell Signaling) was added at 40 µl/well to the treated plate. After the reaction was carried out at 30°C for 1 hour, the plate was washed 5 times, the 1,000-fold diluted anti-pBadser112 rabbit antibody (Cell Signaling) was added at 100 µl/well, and the reaction was carried out at 37°C for 1 hour. After the plate was washed 5 times, the 10,000-fold diluted alkaline phosphatase-labeled anti-rabbit immunoglobulin antibody was added at 100 µl/well, and the reaction was carried out at 37°C for an additional 1 hour. After the plate was washed 10 times, a substrate solution (pH 9.8, 0.1% para-nitrophenyl phosphate (Wako), 1 mol/l diethanolamine (Wako)) was added at 100 µl/well, the reaction was carried out at room temperature for 20 to 30 minutes, and a reaction terminator (3N NaOH (Wako)) was added at 100 µl/well to terminate the reaction. The plate was subjected to the absorbance assay at 405 nm using a microplate reader (Model 550, Bio-Rad). As a wash buffer, 0.05% Tween/PBS(-) was used, and the plate was washed with a microplate washer (Model 1575 Immuno Wash, Bio-Rad).

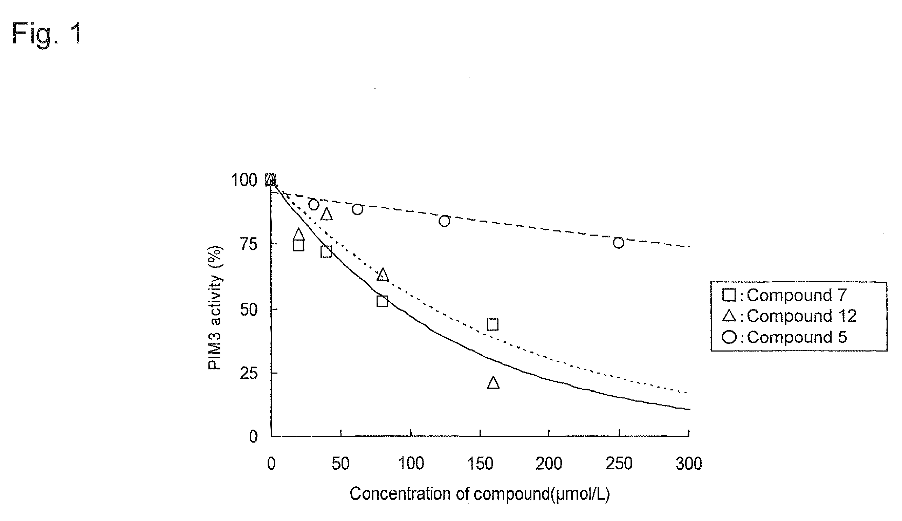

The results are shown in Fig. 1. Compounds 5, 7, and 12 were found to inhibit the Pim-3 activity in a concentration-dependent manner.

### (Example 12) Cell growth assay (pancreatic cell)

Human pancreatic cancer cell lines (PCI35, PCI55, PCI66, MiaPaCa-2, L3.6pl, and PANC-1) were dispensed into a 96-well cell culture Microtest Plate (Becton Dickinson) at 3,000 cells/well with 100 µl of culture media. The media were removed 16 to 18 hours after the initiation of culture, and media containing the test compounds (i.e., 0.1% DMSO, Compound 7 (4, 6, 8, 10 µmol/l), Compound 12 (5, 10, 20, 30 µmol/l)) were added at 100 µl/well. After culture was continued for 0, 24, 48, 72, and 96 hours, the medium containing the test compound was removed, and a 10% WST-1 medium (Roche) was added at 100 µl/well. After culture was conducted for an additional 1 hour, the absorbance at 450 nm was assayed using a microplate reader (Model 550, Bio-Rad). RPMI-1640 medium (SIGMA) was used for the PCI35, PC155, PCI66, MiaPaCa-2, and PANC-1 cell lines, MEM medium (Invitrogen) was used for the L3.6pl cell lines, and 10% calf serum (Invitrogen), 50 U/ml penicillin G (SIGMA), and 50 µg/ml streptomycin (SIGMA) were added to every medium. The cell lines were cultured in a 37°C 5% CO₂ carbon dioxide incubator (ESPEC).

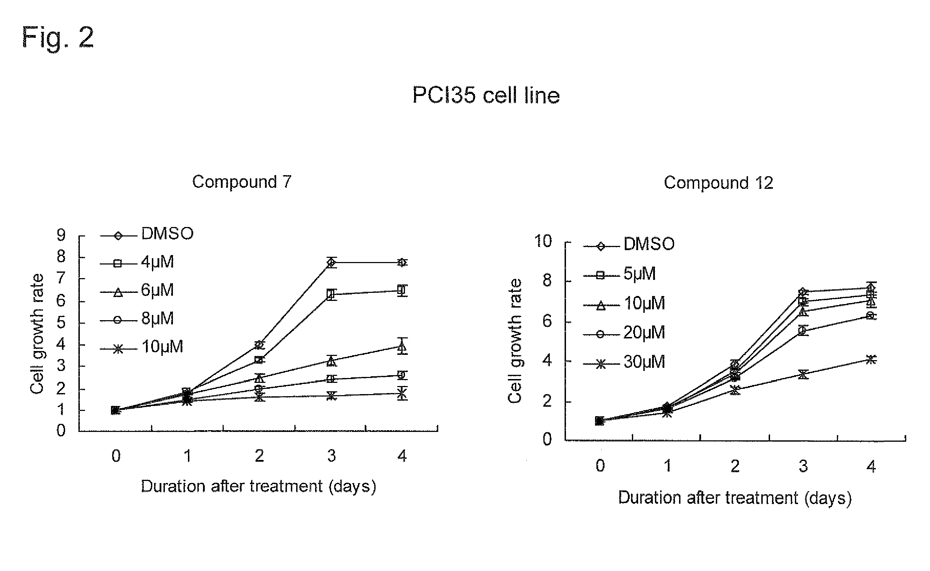

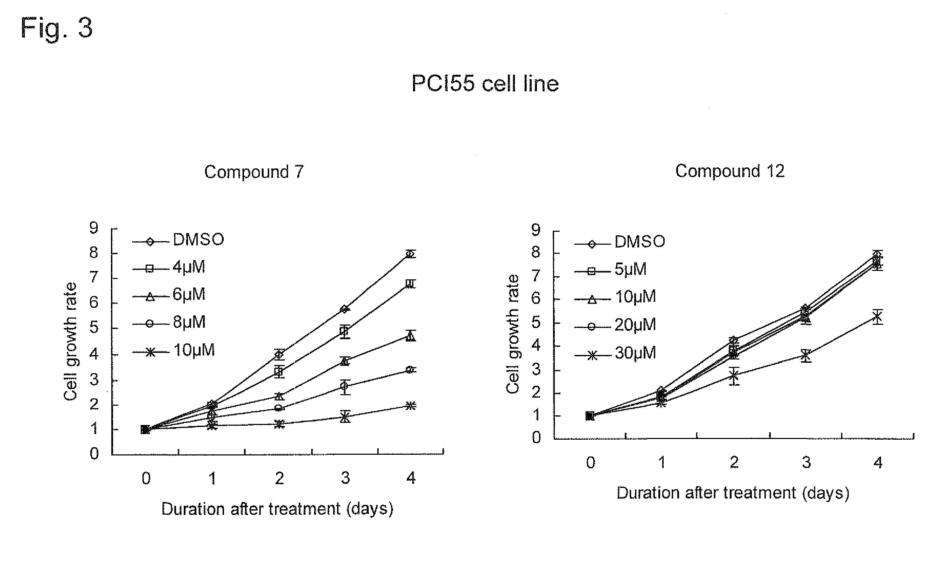

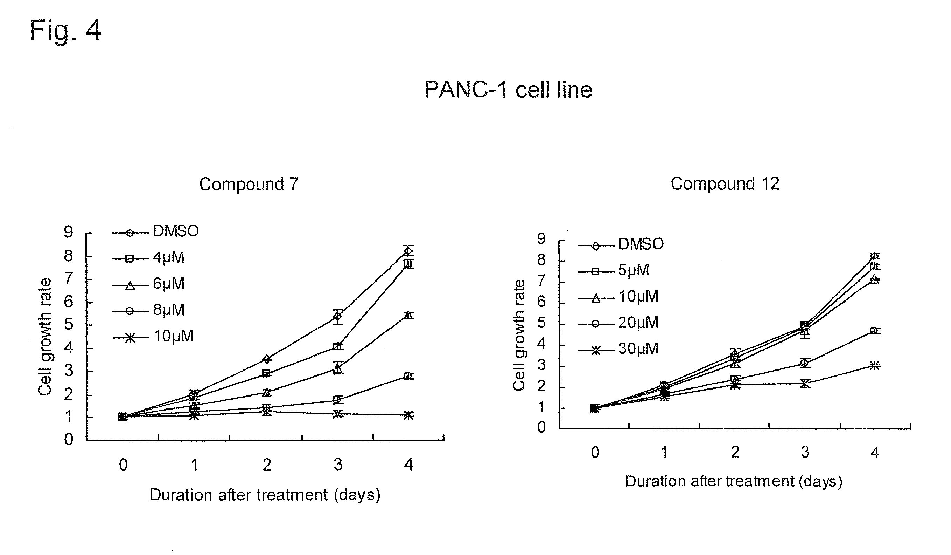

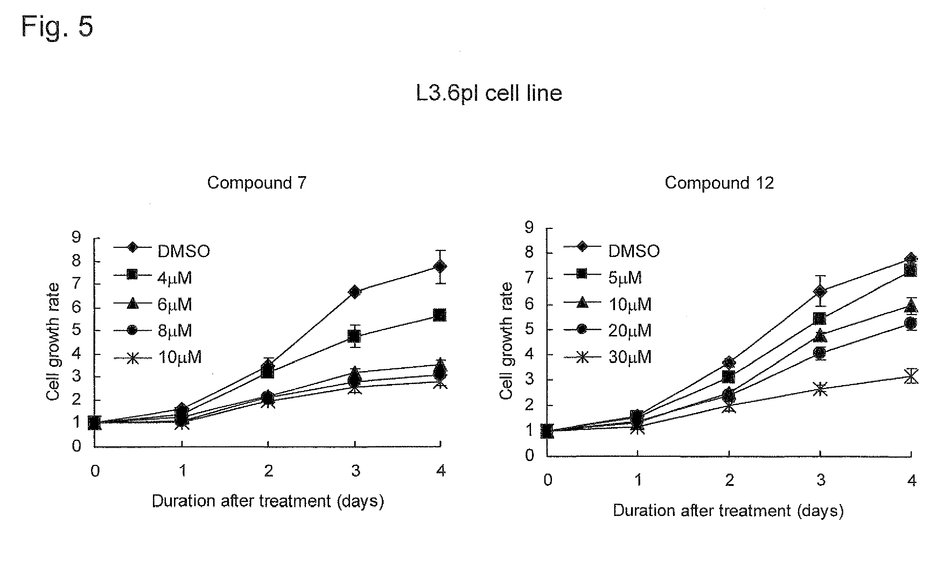

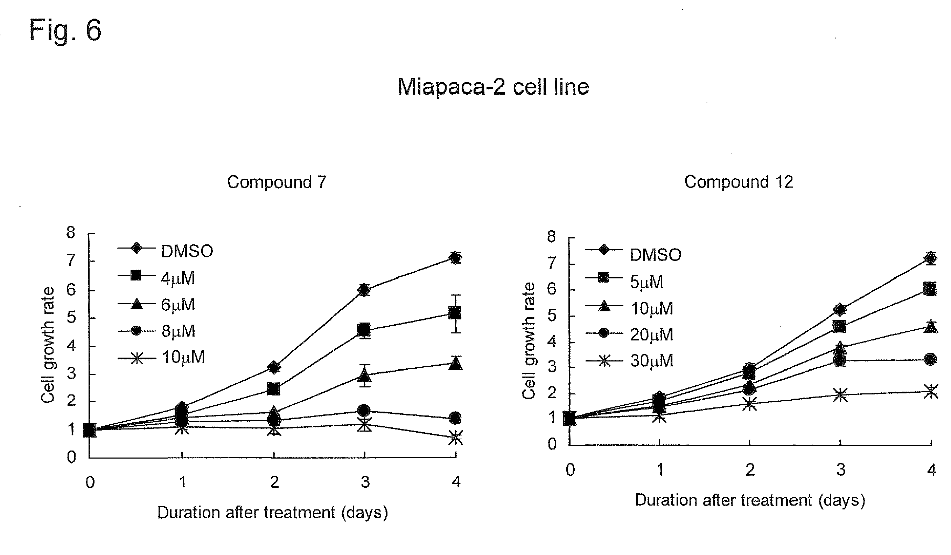

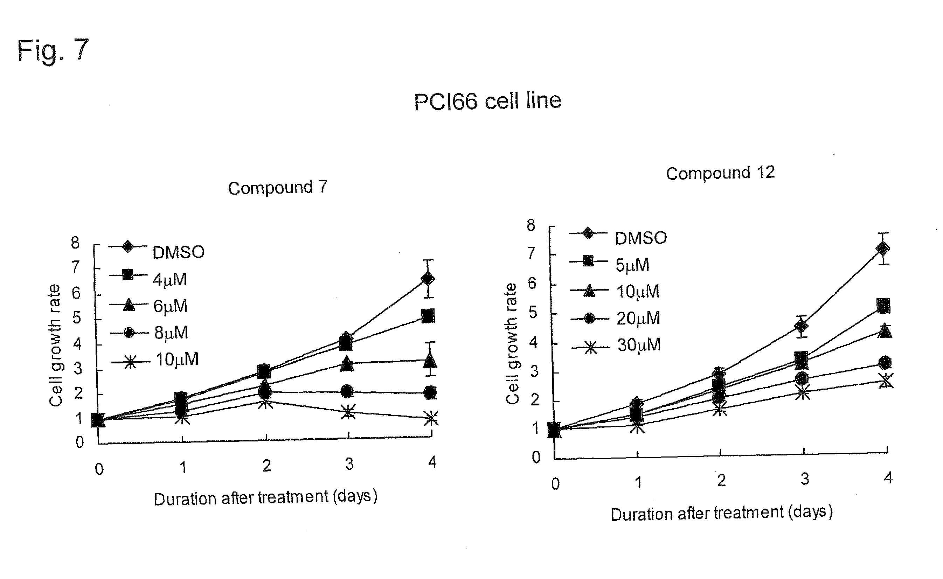

The results are shown in Fig. 2 to Fig. 7. Compounds 7 and 12 were found to inhibit the growth of all tested human pancreatic cancer cell lines (PCI35, PCI55, PCI66, MiaPaCa-2, L3.6pl, and PANC-1).

### (Example 13) Cell growth assay (liver cancer cell)

Human liver cancer cell lines (Hep3B, HuH7, and HepG2 at 4000, 3000, and 4000 cells/well) were dispensed into a 96-well cell culture Microtest Plate (Becton Dickinson) with 100 µl of culture media. The media were removed 16 to 18 hours after the initiation of culture, and media containing the test compounds (0.1% DMSO, Compound 7 (4, 6, 8, and 10 µmol/l) were added at 100 µl/well. After culture was continued for 0, 24, 48, 72, and 96 hours, the medium containing the test compound was removed, and a 10% WST-1 medium (Roche) was added at 100 µ/well. After culture was conducted for an additional 1 hour, the absorbance at 450 nm was assayed using a microplate reader (Model 550, Bio-Rad). RPMI-1640 medium (SIGMA) was used, and 10% calf serum (Invitrogen), 50 U/ml penicillin G (SIGMA), and 50 µg/ml streptomycin (SIGMA) were added to the medium. The cell lines were cultured in a 37°C 5% CO₂ carbon dioxide incubator (ESPEC).

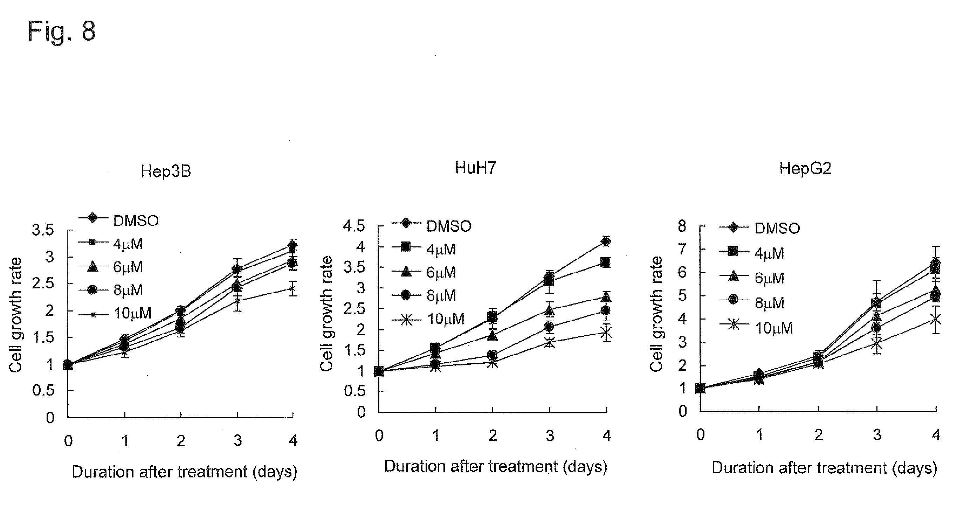

The results are shown in Fig. 8. Compound 7 was found to inhibit the growth of all tested human liver cancer cell lines (Hep3B, HuH7, and HepG29).

### (Example 14) Cell growth assay (colon cancer cell)

Human colon cancer cell lines (SW48, SW480, and HT29 at 3,000 cells/well) were dispensed into a 96-well cell culture Microtest Plate (Becton Dickinson) with 100 µl of culture media. The media were removed 16 to 18 hours after the initiation of culture, and the media containing the test compounds (0.1% DMSO, Compound 7 (4, 6, 8, and 10 µmol/l)) were added at 100 µl/well. After culture was continued for 0, 24, 48, 72, and 96 hours, the medium containing the test compound was removed, and a 10% WST-1 medium (Roche) was added at 100 µl/well. After culture was conducted for an additional 1 hour, the absorbance at 450 nm was assayed using a microplate reader (Model 550, Bio-Rad). RPMI-1640 medium (SIGMA) was used, and 10% calf serum (Invitrogen), 50 U/ml penicillin G (SIGMA), and 50 µg/ml streptomycin (SIGMA) were added to the medium. The cell lines were cultured in a 37°C 5% CO₂ carbon dioxide incubator (ESPEC).

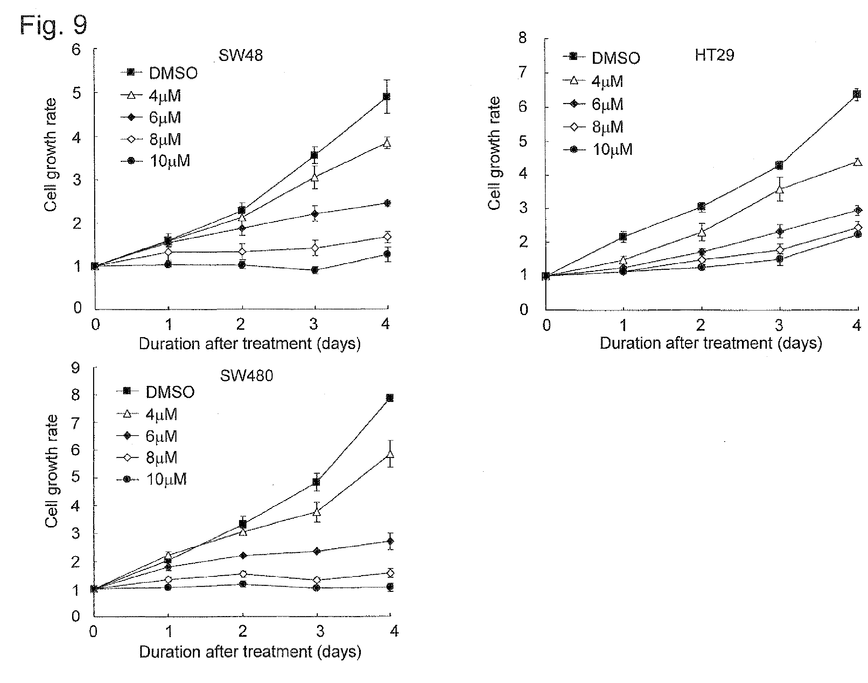

The results are shown in Fig. 9. Compound 7 was found to inhibit the growth of all tested human colon cancer cell lines (SW48, SW480, and HT29).

All publications, patents, and patent applications cited herein are incorporated herein by reference in their entirety.

## Claims

1. A compound according to formula I: 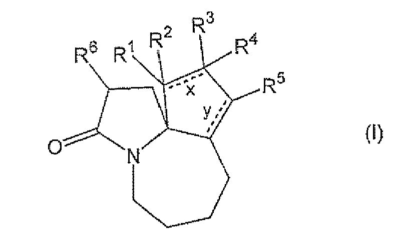 wherein
R¹ and R² are independently H, halogen, C₁₋₆ haloallCyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R¹ and R², together with the carbon atom to which they are attached, may form an optionally substituted 4-, 5-, or 6-membered ring or a carbonyl group (>C=O);
R³ and R⁴ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d} -NR^{b}R^{c}, -R^{a}NR^{b}R^{c}, -R^{a}C(O)R^{d} -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R³ and R⁴ may together form =CH-(CH₂)ₙ-Ar, =CH-R^{a}Ar, =CH-(CH₂)ₙ-Het, =CH-R^{a}Het, or an oxo group (=O);
R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
a broken line indicates that a double bond may be present and, when a double bond represented by x is present, either R¹ or R² and either R³ or R⁴ are absent;
n is independently 0, 1, or 2;
m is independently 0, 1, or 2;
R^{a} is independently C₁₋₆ alkylene, C₃₋₁₀ cycloalkylene, C₂₋₆ alkenylene, C₃₋₁₀ cycloalkenylene, or C₂₋₆ alkynylene;
R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R³C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)ₘR^{d};
R^{d} is independently H, C₁₋₆ alkyl, C₂₋₆ alkenyl, C₂₋₆ alkynyl, C₃₋₁₀ cycloalkyl, or -Ar; R^{e} and R^{f} are independently H or C₁₋₆ alkyl;
Ar is independently optionally substituted aryl; and
Het is independently an optionally substituted 4-, 5-, or 6-membered heterocyclic group,
except for a case in which R¹ and R², together with the carbon atom to which they are attached, form a 5-membered ring according to the following formula: 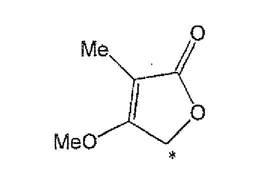 wherein * represents the carbon to which R¹ and R² are attached, R³ and R⁴ together form an oxo group (=O), R⁵ is a methyl group, a double bond represented by y is present, and R⁶ is H, or a salt, solvate or physiologically functional derivative thereof.

2. The compound according to claim 1 or a salt, solvate or physiologically functional derivative thereof,
wherein
R¹ is OH or H and R² is -R^{a}CO₂R^{d} or
R¹ and R², together with the carbon atom to which they are attached, form a carbonyl group (>C=O) or a 5-membered ring according to formula II: 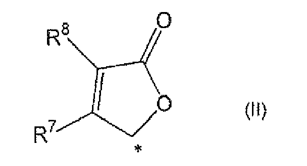 wherein R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{C}, -R^{a}C(O)R^{d}, -C(O)R^{d}, -CO₂R^{d}, -R^{a}CO₂R^{d}, -C(O)NR^{b}R^{e}, -C(O)Ar, -C(O)Het, -S(O)₂NR^{b}R^{c}, -S(O)ₘR^{d}, -S(O)ₘAr, cyano, nitro, or azide;
* represents the carbon to which R¹ and R² are attached;
R³ and R⁴ are both H, or
R³ and R⁴ together form =CH-(CH₂)ₙ-Ar or an oxo group (=O),
provided that, when a double bond represented by x is present, R¹ and R³ are absent; R⁵ is H or C₁₋₆ alkyl;
R⁶ is H; and
R^{a} to R^{d}, n, and m are as defined in claim 1.

3. The compound according to claim 2 or a salt, solvate or physiologically functional derivative thereof,
wherein
R¹ is OH or H and R² is -C = C-CO₂-C₁₋₆ alkyl, or
R¹ and R², together with the carbon atom to which they are attached, form a carbonyl group (>C=O) or a 5 -membered ring according to formula II: 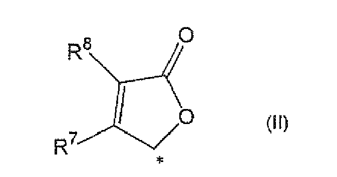 wherein
R⁷ is C₁₋₆ alkoxy and R⁸ is C₁₋₆ alkyl or halogen;
R³ and R⁴ are both H, or
R³ and R⁴ together form =CH-phenyl, -CH-halophenyl, or an oxo group (=O), provided that, when a double bond represented by x is present, R¹ and R³ are absent; R⁵ is H or C₁₋₆ alkyl;
R⁶ is H; and
R^{a} to R^{d} and m are as defined in claim 1.

4. A compound selected from the group consisting of the compounds below or a salt, solvate or physiologically functional derivative thereof.

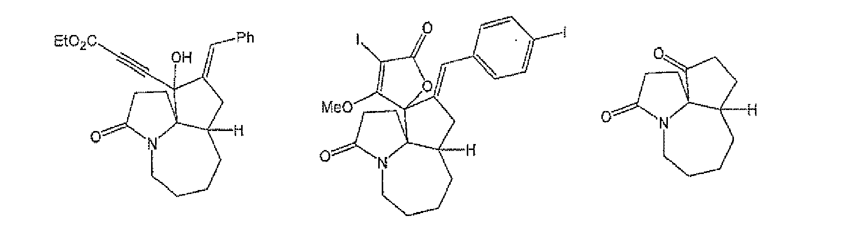

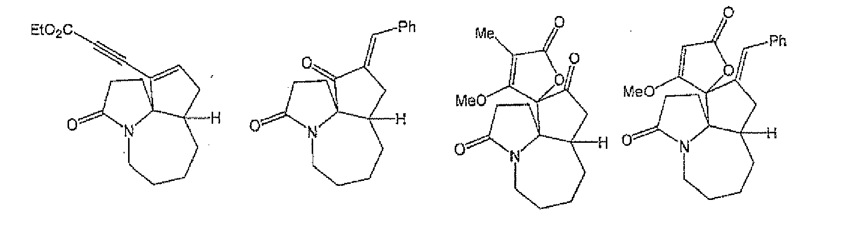

5. A pharmaceutical composition comprising the compound according to any one of claims 1 to 4 or a salt, solvate or physiologically functional derivative thereof.

6. A pharmaceutical composition for prevention and/or treatment of cancer, which comprises, as an active ingredient, a compound according to formula I : 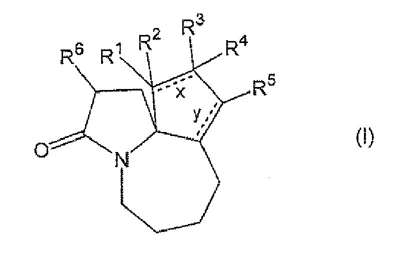 wherein
R¹ and R² are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{e}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{C}, -S(O)ₘR^{d}, -S(O)ₘAr, cyano, nitro, or azide, or
R¹ and R², together with the carbon atom to which they are attached, may form an optionally substituted 4-, 5-, or 6-membered ring or a carbonyl group (>C=O);
R³ and R⁴ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R³NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide, or
R³ and R⁴ may together form =CH-(CH₂)ₙ-Ar, =CH-R^{a}Ar, =CH-(CH₂)ₙ-Het, =CH-R^{a}Het, or an oxo group (=O);
R⁵ and R⁶ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d} -NR^{b}R^{c} -R^{a}NR^{b}R^{c}, -R^{a}C(O)R^{d}, -C(O)R^{d}, -CO₂R^{d}, -R^{a}CO₂R^{d}, -C(O)NR^{b}R^{c}, -C(O)Ar, -C(C))Het, -S(O)₂NR^{b}R^{c}, -S(O)ₘR^{d}, -S(O)ₘAr, cyano, nitro, or azide;
a broken line indicates that a double bond may be present and, when a double bond represented by x is present, either R¹ or R² and either R³ or R⁴ are absent;
n is independently 0, 1, or 2;
m is independently 0, 1, or 2;
R³ is independently C₁₋₆ alkylene, C₃₋₁₀ cycloalkylene, C₂₋₆ alkenylene, C₃₋₁₀ eycloalkenylene, or C₂₋₆ alkynylene;
R^{b} and R^{c} are independently H, C₁₋₆ alkyl, C₂₋₆ alkenyl, C₂₋₆ alkynyl, C₃₋₁₀ cycloalkyl, C₃₋₁₀ cycloalkenyl, -R³C₃₋₁₀ cycloalkyl, -R^{a}OH, -R^{a}OR^{d}, -R^{a}NR^{e}R^{f}, -Ar, -Het, -R^{a}Ar, -R^{a}Het, or -S(O)ₘR^{d};
R^{d} is independently H, C₁₋₆ alkyl, C₂₋₆ alkenyl, C₂₋₆ alkynyl, C₃₋₁₀ cycloalkyl, or -Ar; R^{e} and R^{f} are independently H or C₁₋₆ alkyl;
Ar is independently optionally substituted aryl; and
Het is independently an optionally substituted 4-, 5-, or 6-membered heterocyclic group,
or a salt, solvate or physiologically functional derivative thereof.

7. The pharmaceutical composition according to claim 6,
wherein
R¹ is OH or H and R² is -R^{a}CO₂R^{d}, or
R¹ and R², together with the carbon atom to which they are attached, form a carbonyl group (>C=O) or a 5-membered ring according to formula II: 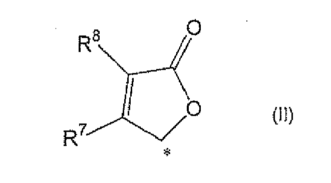 wherein
R⁷ and R⁸ are independently H, halogen, C₁₋₆ haloalkyl, C₁₋₆ alkyl, C₂₋₆ alkenyl, C₂₋₆ alkynyl, C₃₋₁₀ cycloalkyl, C₃₋₁₀ cycloalkenyl, -Ar, -NHAr, -Het, -NHHet, -OR^{d}, -OAr, -OHet, -R^{a}OR^{d}, -NR^{b}R^{c}, -R^{a}NR^{b}R^{C}, -R^{a}C(O)R^{d}, -C(O)R^{d}, -CO₂R^{d}, -R^{a}CO₂R^{d}, -C(O)NR^{b}R^{c}, -C(O)Ar, -C(O)Het, -S(O)₂NR^{b}R^{c}, -S(O)ₘR^{d}, -S(O)ₘAr, cyano, nitro, or azide;
* represents the carbon to which R¹ and R² are attached;
R³ and R⁴ are both H, or
R³ and R⁴ together form =CH-(CH₂)ₙ-Ar or an oxo group (=O),
provided that, when a double bond represented by x is present, R¹ and R³ are absent; R⁵ is H or C₁₋₆ alkyl;
R⁶ is H; and
R^{a} to R^{d}, n, and m are as defined in claim 6.

8. The pharmaceutical composition according to claim 7,
wherein
R¹ is OH or H and R² is -C≡C-CO₂-C₁₋₆ alkyl, or
R¹ and R², together with the carbon atom to which they are attached, form a carbonyl group (>C=O) or a 5-membered ring according to formula II: 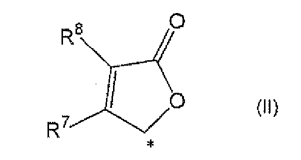 wherein
R⁷ is C₁₋₆ alkoxy and R⁸ is C₁₋₆ alkyl or halogen;
R³ and R⁴ are both H, or
R³ and R⁴ together form =CH-phenyl, =CH-halophenyl, or an oxo group (=O), provided that, when a double bond represented by x is present, R¹ and R³ are absent;
R⁵ is H or C₁₋₆ alkyl;
R⁶ is H; and
R^{a} to R^{d} and m are as defined in claim 6.

9. The pharmaceutical composition according to claim 6, which comprises, as an active ingredient, a compound selected from the group consisting of the compounds below: 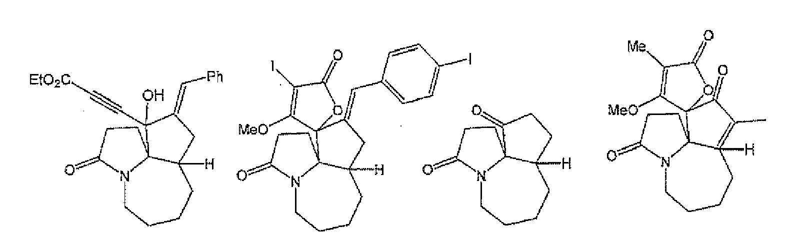 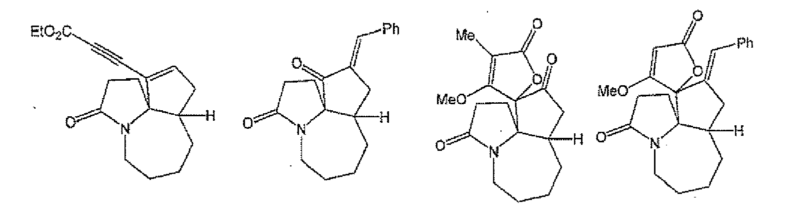 or a salt, solvate or physiologically functional derivative thereof.

10. The pharmaceutical composition according to any one of claims 6 to 9, wherein cancer is pancreatic cancer, gastric cancer, colon cancer, renal cancer, liver cancer, bone marrow cancer, adrenal cancer, skin cancer, melanoma, lung cancer, small intestinal cancer, prostate cancer, testicular cancer, uterine cancer, breast cancer, or ovarian cancer.

11. The pharmaceutical composition according to claim 10, wherein cancer is pancreatic cancer.
